# EUROPEAN PATENT APPLICATION

(11) **EP 4 455 264 A1**
(43) Date of publication of application: **30.10.2024**
(21) Application number: 22911317.0
(22) Date of filing: 21.12.2022
(51) Int. Cl.: C12N 1/21, C12N 1/15, C12N 1/19, C12N 15/31, C12P 19/00

(54) **METHOD FOR PRODUCING SUGAR INCLUDING LACTO-N-TRIOSE II AS CORE TRISACCHARIDE AND METHOD FOR PRODUCING CRYSTALS OF SAID SUGAR**

(30) Priority: 21.12.2021 JP 2021207590
(71) Applicant: Kyowa Hakko Bio Co., Ltd., Tokyo 100-8185 (JP); KIRIN HOLDINGS KABUSHIKI KAISHA, Nakano-ku, Tokyo 164-0001 (JP)
(72) Inventor: SUGITA, Tomotoshi, Tokyo 164-0001 (JP); SANPEI, Sotaro, Tokyo 164-0001 (JP)
(74) Representative: Vossius & Partner Patentanwälte Rechtsanwälte mbB
(86) International application number: PCT/JP2022/047244
(87) International publication number: WO 2023/120615

(57) **Abstract**

The present invention relates to a microorganism having a reduced or inactivated activity of a protein according to any one of the following [1] to [3] and improved productivity of lacto-N-triose II or an oligosaccharide having a lacto-N-triose II (LNTII) skeleton as compared with a parent strain: [1] a protein consisting of the amino acid sequence represented by SEQ ID NO: 2 or 4, [2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and [3] a homologous protein having an oligosaccharide transporting activity and consisting of an amino acid sequence having an identity of 90% or more with the amino acid sequence represented by SEQ ID NO: 2 or 4.

## Description

### TECHNICAL FIELD

The present invention relates to a method for producing a carbohydrate containing lacto-N-triose II as a core trisaccharide, such as lacto-N-triose II, lacto-N-tetraose, or lacto-N-neotetraose, and a method for producing a crystal of the carbohydrate.

### BACKGROUND ART

It has been reported that human milk oligosaccharides (HMO) contained in human breast milk have health functions such as improving the intestinal environment as prebiotics, activating immunity, and developing cognitive functions in infants. In terms of physiological activities thereof, they are expected to be used as an additive in infant formula and as a health functional ingredient for adults (Non Patent Literature 1).

Lacto-N-triose II (hereinafter, referred to as LNTII) is one kind of HMO contained in trace amounts in human colostrum, and is known as an oligosaccharide that constitutes lacto-N-tetraose (hereinafter, referred to as LNT), which is abundantly contained in colostrum (Non Patent Literature 2).

It has been reported that LNTII has strong antibacterial activity to pathogenic Streptococcus and has an immunoregulatory function via TLRs, and that Bifidobacteria can grow using LNTII as a carbon source. The functionalities thereof have attracted attention in recent years among HMOs (Non Patent Literatures 3, 4, and 5).

As a method for producing LNTII or an oligosaccharide containing LNTII as a core trisaccharide, such as LNT, a microbial fermentation method using an N-acetyl glucosamine transferase is widely used. For example, Non Patent Literature 6 discloses a method for producing LNTII or an oligosaccharide containing LNTII as a core trisaccharide, such as lacto-N-neotetraose (hereinafter, referred to as LNnT), by a fermentation method using an N-acetylglucosamine transferase derived from Neisseria meningitides etc. with lactose and UDP-N-acetylglucosamine as a substrate.

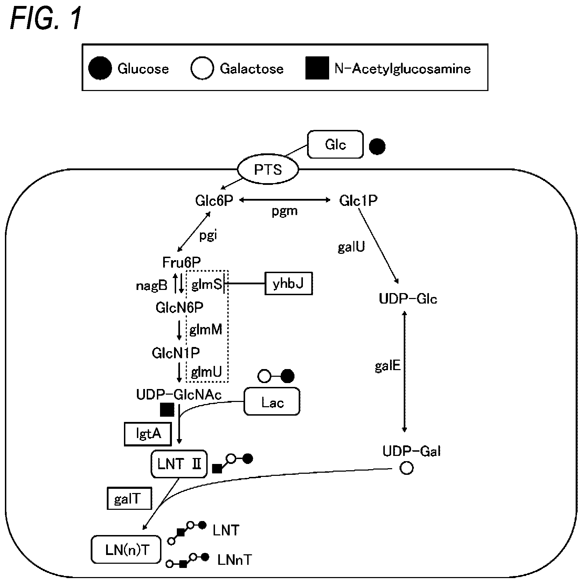

FIG. 1 shows a typical biosynthesis pathway for taking up glucose and synthesizing LNTII, LNT, and LNnT. Glucose is taken up from a culture medium into cells via a phosphoenolpyruvate-dependent phosphotransferase system (PTS). LgtA links N-acetylglucosamine to lactose to produce LNTII. Further, as shown in FIG. 1, LNT or LNnT is produced from LNTII by GalT. LNTII is excreted from cells via LNTII transporters. LNT or LNnT is excreted from cells into the culture medium by oligosaccharide transporters.

As transporters involved in extracellular excretion of LNTII, Patent Literature 1 discloses a YjhB protein derived from Escherichia coli, a ProP protein derived from Mannheimia succiniciproducens, and a SetA protein derived from Cedecea neteri (Patent Literature 1).

Patent Literature 1 also discloses that overexpression of these proteins, which are involved in LNTII transport, increases the production amount of LNTII, and that deletion of a yjhB gene in an Escherichia coli genome prevents the leakage of LNTII and increases the yield of LNT.

As transporters of HMO other than LNTII, a SetA protein and a MdfA protein derived from Escherichia coli and a CDT-2 protein derived from Neurospora crassa, which are involved in excretion of 2-fucosyllactose and 3-fucosyllactose, have been reported (Patent Literatures 2 and 3 and Non Patent Literature 7).

As a method for obtaining an LNTII crystal, a reverse crystallization method has been disclosed in which an LNTII-containing aqueous solution is added to methanol or acetone to obtain an LNTII crystal (Patent Literature 4).

### CITATION LIST

### PATENT LITERATURE

Patent Literature 1: JP2018-526019A
Patent Literature 2: Japanese Patent No. 5580408
Patent Literature 3: WO2021/013708
Patent Literature 4: Japanese Patent No. 4347042

### NON-PATENT LITERATURE

Non Patent Literature 1: Int J Pediatrics (2019) 2390240: 1-8
Non Patent Literature 2: Curr Dev Nutr (2020) 4: nzaa113
Non Patent Literature 3: Acc Chem Res (2019) 52,760
Non Patent Literature 4: J Functional Foods (2019) 59, 174
Non Patent Literature 5: Front Microbiol (2020) 11, 569700
Non Patent Literature 6: Syst Microbiol Biomanufact (2021) 1, 291
Non Patent Literature 7: Metabolic Engineering (2019) 52:232-242

### SUMMARY OF INVENTION

### TECHNICAL PROBLEM

As described above, functionalities of LNTII or an oligosaccharide having an LNTII skeleton and containing LNTII as a core trisaccharide has attracted attention in recent years, and efficient production methods are being sought. Patent Literature 1 also discloses that overexpression of the above-described proteins involved in LNTII transport increases the production amount of LNTII, and deletion of a yjhB gene in an Escherichia coli genome prevents the leakage of LNTII and increases the yield of LNT . However, since some Escherichia coli do not have yjhB or homologous sequences thereof, it is required to search for a new protein that is widely preserved in various Escherichia coli and has an excellent LNTII transporting activity.

In Patent Literatures 2 and 3 and Non Patent Literature 7, as transporters of HMO other than LNTII, a SetA protein and a MdfA protein derived from Escherichia coli and a CDT-2 protein derived from Neurospora crassa, which are involved in excretion of 2-fucosyllactose and 3-fucosyllactose, have been reported. However, it is not known that these proteins have an LNTII transporting activity.

Accordingly, an object of the present invention is to provide a microorganism having excellent productivity of LNTII or an oligosaccharide having an LNTII skeleton and containing LNTII as a core trisaccharide.

A method for efficiently producing an LNTII crystal from a culture solution obtained by the above-described microbial fermentation method is not known. A simple method for purifying and crystallizing high-purity LNTII from a culture solution containing a mixture of various water-soluble substances and a carbohydrate having a structure similar to that of LNTII is required.

Accordingly, an object of the present invention is to provide a method for easily producing a high-purity LNTII crystal.

### SOLUTION TO PROBLEM

The present inventors have found that a microorganism having an enhanced activity of YdeA protein or MdfA protein has improved productivity of LNTII as compared with a parent strain, and completed a first aspect. The present inventors have also found that a microorganism having a reduced or inactivated activity of the proteins has improved productivity of an oligosaccharide having an LNTII skeleton as compared with a parent strain, and completed a second aspect.

Furthermore, the present inventors have completed a method for producing an LNTII crystal using a culture obtained by culturing the microorganism.

That is, the present invention is as follows.
1. A microorganism having an enhanced activity of a protein according to any one of the following [1] to [3] and having improved productivity of lacto-N-triose II (LNTII) as compared with a parent strain:
   [1] a protein consisting of the amino acid sequence represented by SEQ ID NO: 2 or 4,
   [2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
   [3] a homologous protein having an oligosaccharide transporting activity and consisting of an amino acid sequence having an identity of 90% or more with the amino acid sequence represented by SEQ ID NO: 2 or 4.
2. A microorganism having a reduced or inactivated activity of a protein according to any one of the following [1] to [3] and improved productivity of an oligosaccharide having a lacto-N-triose II (LNTII) skeleton as compared with a parent strain:
   [1] a protein consisting of the amino acid sequence represented by SEQ ID NO: 2 or 4,
   [2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
   [3] a homologous protein having an oligosaccharide transporting activity and consisting of an amino acid sequence having an identity of 90% or more with the amino acid sequence represented by SEQ ID NO: 2 or 4.
3. The microorganism according to the above 2, in which
   the oligosaccharide having the LNTII skeleton is lacto-N-tetraose (LNT) or lacto-N-neotetraose (LNnT).
4. A method for producing an oligosaccharide, the method including:
   preparing the microorganism according to any one of the above 1 to 3; and
   producing an oligosaccharide in a culture using the microorganism.
5. A method for producing an LNTII crystal from a culture obtained by culturing the microorganism according to the above 1., the method including the following steps (i) to (iv):
   (i) a step of centrifuging the culture to obtain a supernatant from which bacteria cells are removed;
   (ii) a step of subjecting the supernatant obtained in the step (i) to cation exchange and anion exchange to obtain a solution from which ions are removed;
   (iii) a step of concentrating the solution obtained in the step (ii) with an evaporator; and
   (iv) a step of cooling the solution obtained in the step (iii) or adding dropwise a poor solvent to the solution obtained in the step (iii) to obtain an LNTII crystal.

### ADVANTAGEOUS EFFECTS OF INVENTION

The microorganism according to the first aspect has an enhanced activity of a specific protein, so that the extracellular excretion of LNTII can be promoted, and the productivity of LNTII can be improved. The microorganism according to the second aspect has a reduced or inactivated activity of the protein, so that the extracellular excretion of LNTII can be prevented, and the productivity of an oligosaccharide having an LNTII skeleton which is produced using LNTII as a substrate can be improved. Accordingly, by using the microorganisms according to the first and second aspects, it is possible to produce LNTII or an oligosaccharide having an LNTII skeleton and containing LNTII as a core trisaccharide more efficiently as compared with the related art.

According to the production method of the present invention, a high-purity LNTII crystal can be easily produced by using a culture obtained by culturing the above-described microorganism.

### BRIEF DESCRIPTION OF DRAWINGS

[FIG. 1] FIG. 1 shows a schematic diagram of a biosynthesis pathway of LNTII, LNT, and LNnT in a microorganism according to one embodiment of the present invention.
[FIG. 2] FIG. 2 shows results of analyzing LNTII contained in a supernatant with a carbohydrate analyzer ICS-6000, after culturing an INTC/pLNTII strain and 12 types of LNTII-producing strains having enhanced expression of each transporter gene, and centrifuging the culture solution after appropriately diluting at the end of the culture.
[FIG. 3] FIG. 3 shows a carbohydrate analysis result of an LNTII crystal obtained in Example 6, in which a vertical axis represents an intensity, and a horizontal axis represents an analysis time.
[FIG. 4] FIG. 4 shows a carbohydrate analysis result of an LNTII crystal obtained in Example 7, in which a vertical axis represents an intensity, and a horizontal axis represents an analysis time.
[FIG. 5] FIG. 5 shows an oak ridge thermal ellipsoid plot (ORTEP) diagram of an LNTII 6.0 hydrate crystal.
[FIG. 6] FIG. 6 shows a carbohydrate analysis result of an LNTII crystal obtained in Example 8, in which a vertical axis represents an intensity, and a horizontal axis represents an analysis time.

### DESCRIPTION OF EMBODIMENTS

### 1. Microorganism having Improved Productivity of LNTII

Examples of a microorganism having improved productivity of LNTII of the present invention include the following microorganism.

A microorganism having an enhanced activity of a protein according to any one of the following [1] to [3], and improved productivity of LNTII as compared with a parent strain:
[1] a protein consisting of the amino acid sequence represented by SEQ ID NO: 2 or 4,
[2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
[3] a homologous protein having an oligosaccharide transporting activity and consisting of an amino acid sequence having an identity of 90% or more with the amino acid sequence represented by SEQ ID NO: 2 or 4.

In the present description, the mutant protein refers to a protein obtained by artificially deleting or substituting an amino acid residue of an original protein or artificially inserting or adding an amino acid residue in the protein.

The expression "an amino acid is deleted, substituted, inserted, or added" in the mutant protein of the above [2] may mean that 1 to 20 amino acids are deleted, substituted, inserted, or added at any position in the same sequence. The number of amino acids to be deleted, substituted, inserted, or added is 1 to 20, preferably 1 to 10, further preferably 1 to 8, and most preferably 1 to 5.

An amino acid to be deleted, substituted, inserted, or added may be natural or non-natural. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

In the present description, the homologous protein is a protein for which a gene encoding the protein is thought to have the same evolutionary origin as a gene encoding an original protein due to similarity in structure and function with the original protein. These proteins are possessed by organisms existing in nature.

Examples of the homologous protein include those with an amino acid sequence having an identity of preferably 90% or more, and particularly preferably 95% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, parameters are, for example, Score = 100 and wordlength = 12. When an amino acid sequence is analyzed by BLASTX based on BLAST, the parameters are, for example, score = 50 and wordlength = 3. When BLAST program and Gapped BLAST programs are used, default parameters for each program are used. A specific method of the analysis methods is known.

In the present description, the oligosaccharide transporting activity refers to an activity of transporting an intracellular oligosaccharide to the outside of the cell. The fact that the protein has the oligosaccharide transporting activity can be confirmed by preparing a recombinant DNA containing a DNA encoding the protein by a method to be described later, culturing a microorganism obtained by transforming a microorganism whose oligosaccharide transporting activity cannot be confirmed with the recombinant DNA, preparing a cell extract containing the protein from the obtained culture, appropriately diluting the culture solution, centrifuging the culture solution, and analyzing an oligosaccharide contained in a supernatant with a carbohydrate analyzer. For example, when Escherichia coli W3110 strain is used as the microorganism whose oligosaccharide transporting activity cannot be confirmed, an ability to produce LNTII is artificially imparted during transformation by a method to be described later.

In the present description, the "productivity" refers to an ability to accumulate an object oligosaccharide produced by a microorganism in a culture. The productivity of an oligosaccharide by a microorganism can be confirmed by detecting an oligosaccharide in a culture of the microorganism using a carbohydrate analyzer to be described later.

In the present description, a parent strain refers to an original strain to be subjected to genetic modification, transformation, and the like. In particular, a parent strain of a microorganism having improved productivity of LNTII of the present invention refers to a strain before genetic modification and transformation and the like to enhance the activity of the protein according to any one of [1] to [3].

In the present description, the parent strain is preferably a prokaryote or a yeast strain, more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and most preferably a prokaryote such as Escherichia coli MG1655, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 codon plus (manufactured by Stratagene Corporation), Escherichia coli W3110S3GK (NBRC114657), Serratia ficaria, Serratia fonticola, Serratia liquefaciens, Serratia marcescens, Bacillus subtilis, Bacillus amyloliquefaciens, Brevibacterium immariophilum ATCC14068, Brevibacterium saccharolyticum ATCC14066, Cory nebacterium ammoniagenes, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The parent strain may be a wild-type strain as long as it is a microorganism that produces LNTII, or may be a breeding strain artificially endowed with the ability to produce LNTII when the wild-type strain does not have the ability to produce LNTII.

Examples of a method for artificially endowing the ability to produce LNTII to a microorganism include the following methods (1a) to (1e), and these methods can be used alone or in combination.
(1a) A method of alleviating or releasing at least one mechanism for controlling a biosynthesis pathway for producing LNTII.
(1b) A method for enhancing expression of at least one enzyme associated with the biosynthesis pathway for producing LNTII.
(1c) A method of increasing the number of copies of at least one enzyme gene associated with the biosynthesis pathway for producing LNTII.
(1d) A method of weakening or blocking at least one metabolic pathway branching off from the biosynthesis pathway for producing LNTII to a metabolic product other than a target substance.
(1e) A method of selecting a cell strain having resistance to an LNTII analog higher as compared with a wild-type strain.

The fact that the microorganism is a microorganism capable of producing LNTII can be confirmed by transforming the microorganism with a recombinant DNA containing a DNA encoding a protein consisting of the amino acid sequence represented by SEQ ID NO: 2 or 4, culturing the transformed microorganism in a culture medium, and detecting LNTII accumulated in a culture using a carbohydrate analyzer to be described later.

The parent strain preferably has at least one activity selected from a lactose permease (hereinafter, referred to as lacY) activity, a β1,3-N-acetylglucosamine transferase (hereinafter, referred to as LgtA) activity, a glutamine fructose-6-phosphate transaminase (hereinafter, referred to as ghnS) activity, a phosphoglucosamine mutase (hereinafter, referred to as ghnM) activity and an N-acetylglucosamine-1-phosphate uridyltransferase/glucosamine-1-phosphate acetyltransferase (hereinafter, referred to as gImU) activity, and more preferably has an enhanced activity thereof. Among them, the parent strain preferably has lacY and LgtA activities, and more preferably has enhanced activities thereof.

lacY is a membrane protein that takes up lactose, which is a substrate of LNTII, into cells. As shown in FIG. 1, LgtA is an enzyme associated with production of LNTII from lactose and uridine diphosphate-N-acetylglucosamine (hereinafter, referred to as UDP-GlcNAc). As shown in FIG. 1, glmS, glmM, and ghnU are enzymes associated with the biosynthesis pathway for producing LNTII.

Accordingly, in one embodiment of the present invention, the parent strain is preferably a genetically modified microorganism containing at least one nucleotide sequence selected from the nucleotide sequence encoding lacY (Accession No. BAE76125.1), the nucleotide sequence encoding LgtA (SEQ ID NO: 5), the nucleotide sequence encoding glmS (Accession No. BAE77559.1), the nucleotide sequence encoding glmM (Accession No. BAE77220.1), and the nucleotide sequence encoding glmU (Accession No. BAE77558.1). In particular, the parent strain is more preferably a genetically modified microorganism containing the nucleotide sequence encoding lacY and the nucleotide sequence encoding LgtA. In one embodiment of the present invention, the genetically modified microorganism preferably has an enhanced LNTII-producing ability as compared with a parent strain that is not genetically modified.

A known method may be used as a method for producing Escherichia coli having at least one activity selected from a lacY activity, a LgtA activity, a glmS activity, a glmM activity, and a glmU activity or having an enhanced activity thereof. Specific examples include methods using various genetic manipulations (Syst Microbiol Biomanufact, 2021, 1,291).

In the parent strain, a β-galactosidase (hereinafter, referred to as lacZ) activity and/or a regulatory factor YhbJ activity is preferably reduced or deleted. LacZ is an enzyme that hydrolyzes lactose, which is a substrate of LNTII. Therefore, a decrease in lactose supply can be prevented by losing the lacZ activity. As shown in FIG. 1, YhbJ is a negative transcription regulatory factor of the glmS gene of the LNTII biosynthesis pathway. Therefore, LNTII biosynthesis can be promoted by losing the YhbJ activity.

Accordingly, in one embodiment of the present invention, the parent strain is preferably a genetically modified microorganism having reduced or deleted lacZ and/or YhbJ activity, and more preferably a genetically modified microorganism not containing the nucleotide sequence encoding lacZ and/or the nucleotide sequence encoding YhbJ. In one embodiment of the present invention, the genetically modified microorganism preferably has an enhanced LNTII-producing ability as compared with a parent strain that is not genetically modified.

A known method may be used as a method for producing Escherichia coli having reduced or lost β-galactosidase activity and/or regulatory factor YhbJ activity. Specific examples include methods using various genetic manipulations (Metabolic Engineering, 2017, 41:23-38).

Examples of the microorganism having an enhanced activity of the protein according to any one of the above [1] to [3] as compared with the microorganism of the parent strain include a microorganism having an increased copy number of the gene as compared with the parent strain, which is obtained by transforming the microorganism of the parent strain with a recombinant DNA containing a DNA encoding the protein.

Examples of the microorganism having an increased copy number of the gene as compared with the parent strain, which is obtained by transforming the microorganism of the parent strain with a recombinant DNA containing a DNA encoding the protein according to any one of the above [1] to [3] include a microorganism having an increased copy number of the gene on a chromosomal DNA by transforming the microorganism of the parent strain with the recombinant DNA containing the DNA encoding the protein according to any one of the above [1] to [3], and a microorganism carrying the above gene outside of a chromosomal DNA as a plasmid DNA.

The DNA encoding the protein according to any one of the above [1] to [3] may be any DNA as long as it encodes a protein having an activity of the protein according to any one of [1] to [3]. Specific examples thereof include one DNA selected from the group consisting of the following [4] to [7].
[4] A DNA encoding the protein according to any one of the above [1] to [3].
[5] A DNA consisting of the nucleotide sequence represented by SEQ ID NO: 1 or 3.
[6] A DNA hybridizing with a DNA consisting of a nucleotide sequence complementary to the nucleotide sequence represented by SEQ ID NO: 1 or 3 under stringent conditions and encoding a homologous protein having an oligosaccharide transporting activity.
[7] A DNA consisting of a nucleotide sequence having an identity of 95% or more, preferably 97% or more, more preferably 98% or more, and most preferably 99% or more with the nucleotide sequence represented by SEQ ID NO: 1 or 3 and encoding a homologous protein having an oligosaccharide transporting activity.

In the above [6], the term "hybridizing" means that a DNA hybridizes to a DNA having a specific nucleotide sequence or a part of the DNA. Accordingly, the DNA having a specific nucleotide sequence or a part thereof is a DNA that can be used as a probe for Northern or Southern blot analysis, or a DNA that can be used as an oligonucleotide primer for PCR analysis.

Examples of the DNA used as a probe include DNAs having at least 100 bases or more, preferably 200 bases or more, and more preferably 500 bases or more. Examples of the DNA used as a primer include DNAs having at least 10 bases or more, and preferably 15 bases or more.

A method for a DNA hybridization experiment is well known, and for example, those skilled in the art can determine hybridization conditions according to the present description. The hybridization conditions can be determined according to those described in Molecular Cloning, 4th Edition (2012), Methods for General and Molecular Bacteriology, ASM Press (1994), and Immunology methods manual, Academic press (1996) as well as many other standard textbooks.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mmol/L sodium chloride, 75 mmol/L sodium citrate), 50 mmol/L sodium phosphate (pH 7.6), a 5× Denhardt's solution, 10% dextran sulfate, and a 20 µg/l of denatured salmon sperm DNA, and then the filter is washed in a 0.2×SSC solution at, for example, about 65°C.

Examples of the DNA capable of hybridizing under the above stringent conditions include a DNA having an identity of at least 95% or more, preferably 97% or more, more preferably 98% or more, and most preferably 99% or more with the DNA consisting of the nucleotide sequence represented by SEQ ID NO: 1 or 3 when calculated based on the parameters or the like using BLAST, FASTA, or the like.

The DNA encoding the protein of the above [1] and the DNA of the above [5] can be obtained by, for example, Southern hybridization for a chromosomal DNA library of a microorganism, preferably a microorganism belonging to the genus Escherichia coli, and more preferably an Escherichia coli W3110 strain using a probe DNA that can be designed based on the nucleotide sequence represented by SEQ ID NO: 1 (ydeA gene) or the nucleotide sequence represented by SEQ ID NO: 3 (mdfA gene), or PCR [PCR protocols, Academic Press (1990)] using, as a template, a chromosomal DNA of a microorganism using a primer DNA that can be designed based on the nucleotide sequence.

The Escherichia coli W3110 strain can be available from the National Institute of Technology and Evaluation (NITE) Biological Resource Center.

The DNA encoding the mutant protein of the above [2] can be obtained, for example, by subjecting a DNA consisting of the nucleotide sequence represented by SEQ ID NO: 1 or 3 to error-prone PCR or the like as a template.

Alternatively, the DNA of the above [2] can also be obtained by PCR [Gene, 77, 51 (1989)] using a set of PCR primers each having a nucleotide sequence designed to insert a target mutation (deletion, substitution, insertion, or addition) at the 5' end.

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers each having a 15-base overlap on the 5' side is designed using a plasmid containing a nucleotide sequence designed to introduce a desired mutation (deletion, substitution, insertion, or addition) as a template. At this time, the overlap portion includes a desired mutation. Next, PCR is performed using the mutagenesis primers and using a plasmid containing a nucleotide sequence into which a desired mutation is introduced as a template. When the amplified fragment thus obtained is transformed into Escherichia coli, a plasmid containing a nucleotide sequence into which a desired mutation is introduced can be obtained.

The DNA encoding the homologous protein of the above [3] and the DNA of the above [6] and [7] can be obtained, for example, by a method same as the above method for obtaining the DNA, for example, by searching various gene sequence databases for a nucleotide sequence having an identity of 95% or more, preferably 97% or more, further preferably 98% or more, and most preferably 99% or more with the nucleotide sequence represented by SEQ ID NO: 1 or 3, searching various protein sequence databases for an amino acid sequence having an identity of 95% or more, preferably 97% or more, further preferably 98% or more, and most preferably 99% or more with the amino acid sequence represented by SEQ ID NO: 2 or 4, and using a probe DNA or a primer DNA that can be designed based on the nucleotide sequence or the amino acid sequence obtained by the search, and a microorganism having the DNA.

A nucleotide sequence of the obtained DNA according to any one of the above [4] to [7] can be determined by using the DNA as it is or cleaving the DNA with an appropriate restriction enzyme or the like, incorporating the DNA into a vector by an ordinary method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a 3700 DNA analyzer (manufactured by Applied Biosystems).

The host cell that can be used for determining the nucleotide sequence of the DNA may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990.

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial-length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

A recombinant DNA containing the DNA encoding the protein according to any one of the above [1] to [3] refers to a recombinant DNA in which the DNA is incorporated into an expression vector which is autonomously replicable in a parent strain or can be incorporated into a chromosome and contains a promoter at a position where the DNA can be transferred.

When the recombinant DNA is a recombinant DNA capable of being incorporated into a chromosome, the recombinant DNA may not contain a promoter.

The microorganism having an increased copy number of the gene as compared with the parent strain, which is obtained by transforming the microorganism of the parent strain with the recombinant DNA containing the DNA encoding the protein according to any one of the above [1] to [3] can be obtained by the following method.

Based on the DNA encoding the protein according to any one of the above [1] to [3] and obtained by the above method, a DNA fragment of an appropriate length containing a portion encoding the protein is prepared as necessary. A transformant having an improved production rate can be obtained by substituting a base such that a nucleotide sequence of the portion encoding the protein is an optimal codon for expression in a host cell.

A recombinant DNA is prepared by inserting the DNA fragment downstream of a promoter of an appropriate expression vector. By transforming the parent strain with the recombinant DNA, a microorganism having an increased copy number of a gene encoding the protein as compared with the parent strain can be obtained.

When a prokaryote such as bacteria is used as parent strain, the recombinant DNA is preferably a recombinant DNA composed of a promoter, a ribosomal binding sequence, the DNA according to any one of the above [4] to [7], and a transcription termination sequence. A gene for regulating the promoter may be contained.

It is preferable to use a plasmid in which a distance between a Shine-Dalgarno sequence, which is a ribosomal binding sequence, and an initiation codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

An expression level of the protein having an oligosaccharide transporting activity can be improved by substituting a base such that the nucleotide sequence of the portion encoding the protein having an oligosaccharide transporting activity is an optimal codon for host expression. Examples of the protein having an oligosaccharide transporting activity include the protein according to any one of the above [1] to [3]. Information on the frequency of codon use in the parent strain used in the production method of the present invention can be obtained through a public database.

The expression vector is not particularly limited as long as it is an appropriate nucleic acid molecule for introducing the target DNA into a host and causing the target DNA to be amplified and expressed. Not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW119 (manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK (+), pBluescript II KS (-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p6378-6385], pPAC31 (WO1998/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from an Escherichia coli or a phage etc., such as a uspA promoter, a lac promoter, a PL promoter, a PR promoter, or a PSE promoter can be used. Examples thereof include a promoter which is artificially modified in design, such as a promoter with two trp promoters in series, a tac promoter, a trc promoter, a lacT7 promoter, or a letI promoter.

When a microorganism belonging to the genus Corynebacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

When the above expression vector is used, the promoter may be any promoter as long as it functions in cells of microorganisms belonging to the genus Corynebacterium, and for example, a P54-6 promoter [Appl. Microbiol. Biotechnol., 53, 674-679 (2000)] can be used.

When a yeast strain is used as the parent strain, examples of the expression vector include YEp13 (ATCC37115), YEp24 (ATCC37051), YCp50 (ATCC37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP1 promoter.

By inserting the DNA fragment according to any one of the above [4] to [7] downstream of a promoter of an appropriate expression vector, a recombinant DNA to be used in the production method of the present invention can be prepared.

Examples of a method for introducing a recombinant DNA into a parent strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a host cell include a homologous recombination method. Examples of the homologous recombination method include a method using a plasmid for homologous recombination that can be prepared by linking with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6640-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli containing a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the recombinant DNA is introduced into the parent strain as an autonomously replicable plasmid or incorporated into a chromosome of the parent strain can be confirmed by, for example, a method in which a gene originally contained in a chromosomal DNA of a microorganism cannot be amplified, but the gene introduced through transformation can be amplified by PCR using a primer set to confirm an amplification product. The fact that the transcription amount of the DNA or the production amount of the protein encoded by the DNA is increased can be confirmed by a method in which the transcription amount of the gene of the microorganism is measured by Northern blotting or the production amount of the protein by the microorganism is measured by Western blotting, and the transcription amount of the gene of the microorganism or the production amount of the protein by the microorganism is compared with that of a parent strain.

The fact that the microorganism produced by the above method is a microorganism having an enhanced activity of the protein according to any one of the above [1] to [3] as compared with the parent strain can be confirmed by appropriately diluting a culture solution after culturing the microorganism, centrifuging the culture solution, and analyzing LNTII contained in a supernatant with a carbohydrate analyzer, and comparing LNTII with that of the parent strain.

The above microorganism has an enhanced activity of the protein according to any one of the above [1] to [3] compared to the parent strain, so that the extracellular excretion of LNTII can be promoted, and the productivity of LNTII can be improved. Examples of such a microorganism include an INTC/pLNTII strain having enhanced expression of a ydeA gene or a mdfA gene to be described later in Examples.

In a microorganism having an enhanced activity of a YdeA protein or an MdfA protein, which is an example of such a microorganism, a transporter for excreting LNTII is overexpressed, and the LNTII productivity can be improved by increasing the LNTII excretion.

Examples of the microorganism having improved LNTII productivity include a microorganism having an enhanced activity of a protein according to any one of the following [8] to [10], instead of the protein according to any one of the above [1] to [3].
[8] A protein consisting of the amino acid sequence represented by SEQ ID NO: 65 or 67.
[9] A mutant protein having an oligosaccharide transporting activity and consisting of an amino acid sequence in which 1 to 20 amino acids are deleted, substituted, inserted, or added in the amino acid sequence represented by SEQ ID NO: 65 or 67.
[10] A homologous protein having an oligosaccharide transporting activity and consisting of an amino acid sequence having an identity of 90% or more with the amino acid sequence represented by SEQ ID NO: 65 or 67.

### 2. Microorganism having Improved Productivity of Oligosaccharide having LNTII Skeleton

Examples of the microorganism having improved productivity of an oligosaccharide having an LNTII skeleton of the present invention include the following microorganism.

A microorganism having a reduced or inactivated activity of a protein according to any one of the following [1] to [3] and improved productivity of an oligosaccharide having a lacto-N-triose II (LNTII) skeleton as compared with a parent strain:
[1] a protein consisting of the amino acid sequence represented by SEQ ID NO: 2 or 4,
[2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
[3] a homologous protein having an oligosaccharide transporting activity and consisting of an amino acid sequence having an identity of 90% or more with the amino acid sequence represented by SEQ ID NO: 2 or 4.

Examples of the microorganism having a reduced or inactivated activity of the protein according to any one of [1] to [3] as compared with a parent strain include (a) a microorganism whose specific activity of the protein is reduced to 80% or less, preferably 50% or less, more preferably 30% or less, further preferably 20% or less, particularly preferably 10% or less, and most preferably 0% as compared with the parent strain, and (b) a microorganism whose transcription amount of the gene or production amount of the protein is reduced to 80% or less, preferably 50% or less, more preferably 30% or less, further preferably 20% or less, particularly preferably 10% or less, and most preferably 0% as compared with the parent strain, which can be obtained by introducing deletion, substitution, or addition of bases into the nucleotide sequence of the gene encoding the protein according to any one of the above [1] to [3], which does not contain mutations on a chromosomal DNA. More preferably, a microorganism is one in which a part or all of the gene is deleted.

Examples of the method for producing the microorganism having a reduced or inactivated activity of the protein according to any one of [1] to [3] as compared with a parent strain include known methods such as a method for knocking out a specific gene using PCR [Baba T. et al., Mol systems Biol (2006)], a method for using a homologous recombination system of a lambda phage [Proc. Natl. Acad. Sci. USA, 97, 6640-6645 (2000)], a method for inhibiting the expression of a specific gene by RNAi, and a method for introducing mutations into specific genes and promoter regions thereof using mutation agents such as nitrosoguanidine and ultraviolet light.

In the mutant protein of the above [2], the fact that an amino acid is deleted, substituted, inserted, or added is the same meaning as that an amino acid is deleted, substituted, inserted, or added in 1. described above.

It can be confirmed by the method described in 1. that the mutant protein or homologous protein has an oligosaccharide transporting activity.

The oligosaccharide having an LNTII skeleton is preferably a human milk oligosaccharide containing LNTII as a core trisaccharide. The oligosaccharide having a "core trisaccharide" means that the oligosaccharide contains a specific trisaccharide corresponding to a reducing end of a desired oligosaccharide, and in some cases, the oligosaccharide also contains another sugar moiety together with the specific trisaccharide corresponding to a main moiety.

Examples of the oligosaccharide having an LNTII skeleton include LNT, LNnT, lacto-N-fucopentaose I, lacto-N-fucopentaose II, lacto-N-fucopentaose III, lacto-N-fucopentaose V, lacto-N-difucosylhexose I, lacto-N-difucosylhexaose II, lacto-N-sialylpentaose LSTa, LSTb, LSTc, disialyllacto-N-tetraose, and disialyllacto-N-neotetraose. In the present description, the oligosaccharide having an LNTII skeleton does not include LNTII.

In one embodiment of the present invention, the oligosaccharide having an LNTII skeleton is preferably LNT or LNnT, and a precursor thereof is more preferably LNTII.

A parent strain of the microorganism having improved productivity of an oligosaccharide having an LNTII skeleton of the present invention refers to a strain before genetic modification, transformation, or the like to reduce or inactivate the activity of the protein according to any one of the above [1] to [3]. The parent strain may be a wild-type strain as long as it is a microorganism which produces an oligosaccharide having an LNTII skeleton, or may be a breeding strain artificially endowed with the ability to produce LNTII when the wild-type strain does not have the ability to produce LNTII.

Examples of a method for artificially endowing the ability to produce an oligosaccharide having an LNTII skeleton to a microorganism include the following methods (2a) to (2e), and these methods can be used alone or in combination.
(2a) A method of alleviating or releasing at least one mechanism for controlling a biosynthesis pathway for producing an oligosaccharide having an LNTII skeleton.
(2b) A method for enhancing expression of at least one enzyme associated with the biosynthesis pathway for producing an oligosaccharide having an LNTII skeleton.
(2c) A method of increasing the number of copies of at least one enzyme gene associated with the biosynthesis pathway for producing an oligosaccharide having an LNTII skeleton.
(2d) A method of weakening or blocking at least one metabolic pathway branching off from the biosynthesis pathway for producing an oligosaccharide having an LNTII skeleton to a metabolic product other than a target substance.
(2e) A method of selecting a cell strain having resistance to an analog of an oligosaccharide having an LNTII skeleton higher than that of a wild-type strain.

The fact that the microorganism is a microorganism capable of producing an oligosaccharide having an LNTII skeleton can be confirmed by culturing, in a culture medium, a microorganism in which the protein consisting of the amino acid sequence represented by SEQ ID NO: 2 or 4 is reduced or inactivated by a method according to various genetic manipulations, and detecting the oligosaccharide having an LNTII skeleton accumulated in a culture using a carbohydrate analyzer to be described later.

The parent strain preferably has at least one activity of a lacY activity, a LgtA activity, a glmS activity, a ghnM activity and a glmU activity, a β1,3-galactosyltransferase (hereinafter, referred to as GalT) activity, a phosphoglucomutase (hereinafter, referred to as Pgm) activity, a UTP glucose-1-phosphate uridylyltransferase (hereinafter, referred to as galU) activity, a UDP glucose-4-epimerase (hereinafter, referred to as galE) activity, a UTP glucose-1-phosphate uridylyltransferase (hereinafter, referred to as galF) activity, and a glucose-6-phosphate isomerase (hereinafter, referred to as Pgi) activity, and more preferably has an enhanced activity thereof. Among them, the parent strain preferably has lacY, LgtA, and GalT activities, and more preferably has enhanced activities thereof.

lacY, LgtA, ggmS, glmM, and ggmU are the same as the lacY, LgtA, glmS, ghnM, and glmU in 1. described above. GalT is an enzyme responsible for conversion from LNTII to LNT or LNnT. As shown in FIG. 1, Pgm, galU, galE, and galF are enzymes associated with the pathway for producing uridine diphosphate galactose (hereinafter, referred to as UDP-Gal). As shown in FIG. 1, Pgi is an enzyme associated with the pathway for producing LNTII.

Accordingly, in one embodiment of the present invention, the parent strain is preferably a genetically modified microorganism containing at least one nucleotide sequence selected from the nucleotide sequence encoding lacY, the nucleotide sequence encoding LgtA (SEQ ID NO: 5), the nucleotide sequence encoding glmS, the nucleotide sequence encoding glmM, the nucleotide sequence encoding glmU, the nucleotide sequence encoding GalT, the nucleotide sequence encoding Pgm (Accession No. BAA35337.1), the nucleotide sequence encoding galU (Accession No. BAA36104.1), the nucleotide sequence encoding galE (Accession No. BAA35421.1), the nucleotide sequence encoding galF (Accession No. BAA15896.1), and the nucleotide sequence encoding Pgi (Accession No. BAE78027.1). In particular, the parent strain is preferably a genetically modified microorganism containing the nucleotide sequence encoding lacY, the nucleotide sequence encoding LgtA, and the nucleotide sequence encoding GalT.

In one embodiment of the present invention, the genetically modified microorganism preferably has an enhanced ability to produce an oligosaccharide having an LNTII skeleton as compared with a parent strain that is not genetically modified.

A known method may be used as a method for producing Escherichia coli having at least one activity selected from a lacY activity, a LgtA activity, a glmS activity, a glmM activity, a glmU activity, a GalT activity, a Pgm activity, a galU activity, a galE activity, a galF activity, and a Pgi activity, or having an enhanced activity thereof. Specific examples include a method according to various genetic manipulations (Non Patent Literature 6) may be used.

In the parent strain, a β-galactosidase (hereinafter, referred to as lacZ) activity and/or a regulatory factor YhbJ activity is preferably reduced or deleted.

Accordingly, in one embodiment of the present invention, the parent strain is preferably a genetically modified microorganism containing the nucleotide sequence encoding lacZ and/or the nucleotide sequence encoding YhbJ.

In one embodiment of the present invention, the genetically modified microorganism preferably has an enhanced ability to produce an oligosaccharide having an LNTII skeleton as compared with a parent strain that is not genetically modified.

A known method may be used as a method for producing Escherichia coli whose β-galactosidase activity and/or regulatory factor YhbJ activity is lost. Specific examples include a method according to various genetic manipulations (Metabolic Engineering, 2017, 41: 23-38).

The fact that the microorganism produced by the above method is a microorganism having a reduced or inactivated activity of the protein according to any one of the above [1] to [3] as compared with a parent strain can be confirmed by appropriately diluting a culture solution after culturing the microorganism, centrifuging the culture solution, and analyzing LNTII contained in a supernatant with a carbohydrate analyzer, and comparing LNTII with that of the parent strain.

The above-described microorganism has a reduced or inactivated activity of the protein according to any one of the above [1] to [3] as compared with the parent strain, so that the extracellular excretion of LNTII can be prevented, and the productivity of the oligosaccharide having an LNTII skeleton which is produced using LNTII as a substrate can be improved. In a microorganism having a reduced or inactivated activity of a YdeA protein or an MdfA protein, which is an example of such a microorganism, an exporter protein that excretes LNTII is deleted or inactivated, and the productivity of the oligosaccharide having an LNTII skeleton containing LNT and LNnT is improved.

Examples of the microorganism having improved productivity of the oligosaccharide having an LNTII skeleton include a microorganism having a reduced or inactivated activity of a protein according to any one of the following [8] to [10], instead of the protein according to any one of the above [1] to [3].
[8] A protein consisting of the amino acid sequence represented by SEQ ID NO: 65 or 67.
[9] A mutant protein having an oligosaccharide transporting activity and consisting of an amino acid sequence in which 1 to 20 amino acids are deleted, substituted, inserted, or added in the amino acid sequence represented by SEQ ID NO: 65 or 67.
[10] A homologous protein having an oligosaccharide transporting activity and consisting of an amino acid sequence having an identity of 90% or more with the amino acid sequence represented by SEQ ID NO: 65 or 67.

### 3. Method for Producing Oligosaccharide

A method for producing an oligosaccharide of the present invention includes the following methods.

A method for producing an oligosaccharide includes: preparing the microorganism according to any one of the above in 1. and 2.; and producing an oligosaccharide in a culture using the microorganism.

In the method of the present invention, the desired oligosaccharide is preferably a human milk oligosaccharide containing LNT-II as a core trisaccharide. The oligosaccharide having a "core trisaccharide" means that the oligosaccharide contains a specific trisaccharide corresponding to a reducing end of a desired oligosaccharide, and in some cases, the oligosaccharide also contains another sugar moiety together with the specific trisaccharide corresponding to a main moiety.

The oligosaccharide means a sugar polymer composed of at least three moieties, that is, includes trisaccharides, tetrasaccharides, pentasaccharides, etc., and is preferably an oligosaccharide selected from at least one of LNTII, LNT, LNnT, lacto-N-Fucopentaose I, lacto-N-Fucopentaose II, lacto-N-Fucopentaose III, lacto-N-Fucopentaose V, lacto-N-difucosylhexose I, lacto-N-difucosylhexaose II, lacto-N-Sialylpentaose LSTa, LSTb, LSTc, disialyllacto-N-tetraose, and disialyllacto-N-neotetraose. Among them, LNTII, LNT, and LNnT are particularly preferred.

The method for culturing the microorganism according to any one of the above in 1. and 2. can be performed according to a usual method used for culturing a microorganism.

As a culture medium for culturing a microorganism, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

As the microorganism of the present invention used in the method for producing an oligosaccharide, a microorganism having an ability to produce a receptor carbohydrate such as glucose, lactose, or lactose monohydrate may be used.

In the method for producing an oligosaccharide, glucose, lactose, lactose monohydrate, etc. may be added to the culture medium during culture.

In the method for producing an oligosaccharide, instead of adding glucose, lactose, lactose monohydrate, etc. to the culture medium during culture, a microorganism having an ability to produce glucose, lactose, lactose monohydrate, etc. from sugar may be co-cultivated with the microorganism of the present invention to supply glucose, lactose, or lactose monohydrate, etc. to the microorganisms of the present invention.

In the method for producing an oligosaccharide, β-galactosidase and YhbJ are preferably absent in the culture medium.

The culture is generally preferably performed under aerobic conditions such as shaking culture or deep aeration stirring culture. The culture temperature is generally 30°C to 37°C, and the culture time is generally 24 hours to 3 days. The pH of the culture solution during culture is generally maintained at 6.0 to 9.0. The pH is adjusted using an inorganic or organic acid, an alkaline solution, urea, calcium carbonate, ammonia, or the like.

The oligosaccharide can be produced by producing and accumulating the oligosaccharide in the culture by the above-described culture and collecting the oligosaccharide from the culture.

Generally, the oligosaccharide can be collected from a supernatant after centrifugation of the culture. When the oligosaccharide is accumulated in bacterial cells, for example, the oligosaccharide can be collected, by using an ion exchange resin method or the like, from a supernatant obtained by crushing the bacterial cells by ultrasonic waves or the like and removing the bacterial cells by centrifugation.

### 4. Method for Producing LNTII Crystal

A method for producing an LNTII crystal of the present invention includes the following method.

A method for producing an LNTII crystal from a culture obtained by culturing the microorganism according to the above 1. includes the following steps (i) to (iv):
(i) a step of centrifuging the culture to obtain a supernatant from which bacteria cells are removed;
(ii) a step of subjecting the supernatant obtained in the step (i) to cation exchange and anion exchange to obtain a solution from which ions are removed;
(iii) a step of concentrating the solution obtained in the step (ii) with an evaporator; and
(iv) a step of cooling the solution obtained in the step (iii) or adding dropwise a poor solvent to the solution obtained in the step (iii) to obtain an LNTII crystal.

Hereinafter, each step will be described.

### (i) A step of centrifuging the culture to obtain a supernatant from which bacteria cells are removed

The step (i) is a step of centrifuging the culture of the microorganism described above in the 1. to obtain a supernatant from which bacteria cells are removed. The culture is preferably heated at 60°C to 80°C for 30 minutes to 120 minutes after adjusting the pH to preferably 3.0 to 4.0 before centrifugation.

Conditions of the centrifugation are not particularly limited, and are generally preferably 0°C to 30°C, and more preferably 0°C to 10°C, preferably 4000 G to 12000 G, or 6000 rpm to 10000 rpm, and preferably 5 minutes to 30 minutes.

### (ii) A step of subjecting the supernatant obtained in the step (i) to cation exchange and anion exchange to obtain a solution from which ions are removed

The step (ii) is a step of subjecting the supernatant obtained in the step (i) from which bacteria cells are removed to cation exchange and anion exchange to obtain a solution from which ions are removed. The step (ii) may be performed at room temperature or lower, and is preferably performed at a lower temperature.

A cation exchange resin is not particularly limited, various ion exchange resins can be used as appropriate, and a strong acidic cation exchange resin is preferred. The strong acidic cation exchange resin has, for example, a sulfonic acid group in a crosslinked styrene skeleton. More specifically, examples thereof include resins such as DIAION UBK 550 (H⁺ type) (manufactured by Mitsubishi Chemical Group Corporation) and Marathon C (H⁺ type) (manufactured by Dow Chemical).

An anion exchange resin is not particularly limited, various ion exchange resins can be used as appropriate, and a weak basic anion exchange resin is preferred. The weak basic anion exchange resin has, for example, a primary to tertiary amine group in a crosslinked styrene skeleton, or a carboxylic acid in a crosslinked acrylic skeleton. More specifically, examples thereof include resins such as A111S (OH⁻ type) (manufactured by Proline), A845S (OH⁻ type) (manufactured by Proline), and WA30 (OH⁻ type) (manufactured by Mitsubishi Chemical Group Corporation).

In the cation exchange and the anion exchange, one type of cation exchange resin and one type of anion exchange resin may be used. If necessary, a plurality of cation exchange resins and a plurality of anion exchange resins may be used in combination. After the cation exchange, the anion exchange may be performed. Alternatively, the cation exchange may be performed after the anion exchange.

The pH in the cation exchange is not particularly limited, and is generally preferably 0 to 10, and more preferably 0 to 5. The pH in the anion exchange is not particularly limited, and is generally preferably 4 to 14, and more preferably 9 to 14.

A column filled with the cation exchange resin may be any column as long as it is a column used for purifying a chemical substance. When the column is filled with the ion exchange resin, a ratio of a resin layer height/a column inner diameter is preferably selected to be large, and the ratio of a resin layer height/a column inner diameter is more preferably selected to be 3 or more.

A column filled with the anion exchange resin may be any column as long as it is a column used for purifying a chemical substance. When the column is filled with the ion exchange resin, a ratio of a resin layer height/a column inner diameter is preferably selected to be large, and the ratio of a resin layer height/a column inner diameter is more preferably selected to be 3 or more.

In order to pass the supernatant obtained in the step (i) from which bacteria cells are removed through the column filled with the cation exchange resin, for example, the solution may be passed through the column filled with the ion exchange resin from the top of the column, a so-called column bed upper layer, or may be passed through the column from the bottom of the column, a so-called column bed lower layer. More preferably, the solution is passed through the column from the top. As a column passing rate, a space velocity is preferably 5 [1/hour] or less, and more preferably 3 [1/hour] or less.

In order to pass the supernatant obtained in the step (i) from which bacteria cells are removed through the column filled with the anion exchange resin, for example, the solution may be passed through the column filled with the ion exchange resin from the top of the column, a so-called column bed upper layer, or may be passed through the column from the bottom of the column, a so-called column bed lower layer. More preferably, the solution is passed through the column from the top. As a column passing rate, a space velocity is preferably 5 [1/hour] or less, and more preferably 3 [1/hour] or less.

A fraction obtained after the cation exchange and the anion exchange may be filtered. For filtration, an MF membrane (HVLP 06225, manufactured by Merck Co., Ltd.), an MF membrane module (PSP-003, manufactured by Asahi Kasei Corporation), a UF membrane (WLP 06225, manufactured by Merck Co., Ltd.), a UF membrane module (SIP-0013, manufactured by Asahi Kasei Corporation), or a filtration membrane similar to those described above can be used.

### (iii) A step of concentrating the solution obtained in the step (ii) with an evaporator

The step (iii) is a step of concentrating the solution obtained in the step (ii) with an evaporator to obtain a solution containing LNTII with high purity.

In order to obtain the solution containing LNTII with high purity, a concentration step is performed. The concentration step is generally performed under vacuum at a pressure in a range of, for example, 0 mb to 2 mb, and preferably 0 mb to 1.2 mb. The vacuum makes it possible to lower the temperature required for evaporation and to reduce the duration of the concentration step. This can be performed at a temperature in a range of 5°C to 60°C, preferably 5°C to 50°C, and further preferably 5°C to 40°C.

The concentration step can be performed using a one-step evaporator, and a multi-stage evaporator, for example, a two-stage evaporator. The concentration step can be performed continuously.

The purity of LNTII in the solution containing LNTII with high purity is preferably 50% or more, and further preferably 80% or more.

### (iv) A step of cooling the solution obtained in the step (iii) or adding dropwise a poor solvent to the solution obtained in the step (iii) to obtain an LNTII crystal

The step (iv) is a step of obtaining an LNTII crystal by cooling the solution containing LNTII obtained in the step (iii) or by dropping a poor solvent into the solution.

An LNTII crystal is added as a seed crystal to the solution containing LNTII. As the LNTII crystal to be added as a seed crystal, a crystal obtained by the method of the present invention can be used.

The seed crystal may be added before the crystal precipitation step or during the crystal precipitation step, as long as the seed crystal is added before the LNTII crystal precipitates in the solution.

The seed crystal is added such that a concentration in the solution is generally 0.1 mass% to 10 mass%, and preferably 0.1 mass% to 2.0 mass%.

Examples of the method for precipitating an LNTII crystal in the solution include a method for cooling the solution and a method for dropping a poor solvent. One or more of these methods can also be used in combination.

Water can be used as the solvent of the solution in the method for cooling the solution.

In the method for cooling the solution, the temperature of the solution is generally 0°C to 20°C, preferably 5°C to 20°C, and most preferably 5°C to 10°C, and the cooling time is generally 2 hours to 72 hours, preferably 12 hours to 72 hours, and most preferably 24 hours to 72 hours.

A poor solvent refers to a solvent in which a solubility of a chemical substance is low. A mixture of the poor solvent and the solvent may be used. The poor solvent may be preferably ethanol, methanol, n-propanol, isopropyl alcohol, or acetone. When the solution is mixed, the solubility of the substance in the solvent mixture decreases, and the substance is precipitated.

After the LNTII crystal is precipitated as described above and before the step of collecting the precipitated crystal, the precipitated crystal may be further aged for generally 0.5 hours to 12 hours, preferably 2 hours to 12 hours, and most preferably 2 hours to 6 hours.

The term "aged" refers to temporarily stopping the step of precipitating the LNTII crystal and growing the crystal. After the crystal is aged, the step of precipitating the LNTII crystal may be resumed.

The method for collecting an LNTII crystal is not particularly limited, and examples thereof include filtration, pressure filtration, suction filtration, and centrifugation. Further, in order to reduce adhesion of a mother liquor to the crystal and improve the quality of the crystal, the crystal can be appropriately washed after the crystal is collected. A solution used for crystal washing is not particularly limited, and a solution in which one or more selected from water, methanol, ethanol, acetone, n-propanol, and isopropyl alcohol are mixed at any ratio can be used.

The crystal of the present invention can be obtained by drying the obtained wet crystal. That is, the method for producing a crystal of the present invention may further include a step of drying the LNTII crystal.

The drying conditions may be any method as long as the form of the LNTII crystal can be maintained, such as reduced pressure drying, vacuum drying, fluidized bed drying, or ventilation drying.

The drying temperature may be any range as long as the adhering moisture or solution can be removed, and is preferably 80°C or lower, more preferably 60°C or lower, and further preferably 10°C or lower.

By the above method, a high-purity LNTII crystal can be obtained. The crystal of the present invention is preferably an LNTII.6.0 hydrates crystal.

The fact that the crystal of the present invention is a 6.0 hydrate crystal can be confirmed by single crystal X-ray analysis to be described later.

The purity of the obtained LNTII crystal is generally 80% or more, preferably 90% or more, more preferably 97% or more, 98% or more, further preferably 99% or more, and most preferably 99.5% or more.

Examples of the method for analyzing a structure of the obtained LNTII crystal include powder X-ray diffraction and single crystal X-ray diffraction using CuKa as an X-ray source.

In the step (iv), when water is used as the solvent for the step of cooling the solution obtained in the step (iii), as the LNTII crystal obtained by the above method, specifically, it is preferred that an LNTII crystal has a peak at a diffraction angle (2θ) described in the following (i) in powder X-ray diffraction using CuKa as an X-ray source. It is more preferred that an LNTII crystal has a peak at a diffraction angle (2θ) described in the following (ii) in addition to the diffraction angle (2θ) described.
(i) 19.3, 19.6, 8.2, 19.9, 23.1
(ii) 23.6, 21.4, 9.9, 26.1, 25.0

In the step (iv), when the step of dropping the poor solvent into the solution obtained in the step (iii) is selected, in the case of using the poor solvent other than water such as ethanol, methanol, n-propanol, isopropyl alcohol, or acetone, the LNTII crystal obtained by the above method is preferably an LNTII crystal having a peak at a diffraction angle (2θ) described in the following (iii) in powder X-ray diffraction using CuKa as an X-ray source, and more preferably an LNTII crystal having a peak at a diffraction angle (2θ) described in the following (iv) in addition to the diffraction angle (2θ) described in the following (iii).
(iii) 18.6, 19.8, 9.5, 20.9, 21.5
(iv) 26.9, 22.0, 12.7, 8.3, 29.4

Among the above LNTII crystal, an LNTII crystal having a peak at a diffraction angle (2θ) described in the following (i) is preferred, and an LNTII crystal having a peak at a diffraction angle (2θ) described in the following (ii) in addition to the diffraction angle (2θ) described in the following (i) is more preferred.

### [Analysis Example]

### (1) Analysis and Quantification of LNTII or LNT

In Examples, analysis and quantification of LNTII or LNT were performed by the following procedure.

A culture solution containing microorganisms after culture was centrifuged, and a supernatant was collected. LNTII or LNT contained in the supernatant was analyzed by a carbohydrate analyzer ICS-6000 (manufactured by Thermo Fisher Scientific K.K.).

### [Analysis Conditions]

Column: CarboPAC PA10
Column temperature: 25°C
Mobile phase:
   (mobile phase A) water
   (mobile phase B) 500 mmol/L sodium hydroxide
   (mobile phase C) 300 mmol/L sodium acetate
   Mixing ratio of mobile phase A, mobile phase B, and mobile phase C:
      (0 minutes to 18 minutes) gradient from 80:20:0 to 70:20:10
      (18 minutes to 20 minutes) 70:20:10
      (20 minutes to 25 minutes) 80:20:0
      Flow rate: 0.8 mL/min
      Detector: pulsed amperometry detector

### (2) Single Crystal X-ray Structure Analysis

Single crystal X-ray structure analysis was performed using a single crystal X-ray diffractometer Synergy-R (manufactured by Rigaku Corporation), and the measurement was performed according to an instruction manual.

### (3) Powder X-ray Diffraction

Powder X-ray diffraction was performed using a powder X-ray diffractometer (XRD) Ultima IV (manufactured by Rigaku Corporation), and the measurement was performed according to an instruction manual.

### Examples

Examples of the present invention are shown below, but the present invention is not limited to these Examples.

### [Example 1] Construction of Microorganism Used in Production of Lacto-N-triose II (LNTII)

### (1) Acquisition of DNA fragment to be used as marker for gene deletion

PCR was performed using DNAs consisting of the nucleotide sequences represented by SEQ ID NOs: 9 and 10 as a primer set and pCatSac (Appl Environ Microbiol (2013) 79, 3033-3039) as a template to obtain a cat-sacB fragment containing a chloramphenicol-resistant cat gene and a sucrose sensitive sacB gene.

### (2) Construction of Escherichia coli with loss of β-galactosidase (lacZ) activity, lactose permease (lacY) activity, and regulatory factor YhbJ activity

Escherichia coli deficient in a DNA encoding β-galactosidase (hereinafter, referred to as lacZ gene), a DNA encoding lactose permease (hereinafter, referred to as lacY gene), and a DNA encoding a regulatory factor YhbJ protein (hereinafter, referred to as yhbJ gene) was constructed by the following method. Note that lacZ and lacY form an operon on the Escherichia coli genome.

PCR was performed using, as a template, a genomic DNA of the Escherichia coli W3110 strain prepared by an ordinary method and using, as a primer set, DNAs consisting of the nucleotide sequences shown in "Primer set" in Table 1 to amplify each DNA fragment.

**[Table 1]**

| Primer set (SEQ ID NO:) | Amplified DNA fragment | Remarks |
|---|---|---|
| 11 and 12 | lacZ upstream 1 | Sequences of nucleotide sequences represented by SEQ ID NOs: 9 and 11 at 5' ends are complementary |
| 13 and 14 | lacY downstream 1 | Sequences of nucleotide sequences represented by SEQ ID NOs: 10 and 13 at 5' ends are complementary |
| 12 and 15 | lacZ upstream 2 | Sequences of nucleotide sequences represented by SEQ ID NOs: 15 and 16 at 5' ends are complementary |
| 14 and 16 | lacY downstream 2 | |

The lacZ upstream 1 and lacZ upstream 2 contain a region from an initiation codon of the lacZ gene to about 1000 bp upstream of the initiation codon. The lacY downstream 1 and lacY downstream 2 contain a region from about 40 bp to about 1000 bp downstream of a stop codon of the lacY gene.

PCR was performed using, as a template, a mixture of the lacZ upstream 1, the lacY downstream 1, and the cat-sacB fragment at an equimolar ratio and using, as a primer set, DNAs consisting of the nucleotide sequences represented by SEQ ID NOs: 12 and 14 to obtain a DNA (hereinafter, referred to as lacZY::cat-sacB) fragment consisting of a sequence with the cat-sacB fragment inserted in a region around the lacZ and lacY genes.

PCR was performed using, as a template, a mixture of the lacZ upstream 2 and the lacY downstream 2 at an equimolar ratio and using, as a primer set, DNAs consisting of the nucleotide sequences represented by SEQ ID NOs: 12 and 14 to obtain a DNA (hereinafter, referred to as ΔlacZY) fragment consisting of a sequence with the lacZ upstream and the lacY downstream directly linked to each other without lacZY.

The lacZY::cat-sacB fragment was introduced into a W3110S3GK strain (NBRC114657) carrying a plasmid pKD46 containing a gene encoding λ recombinase [Datsenko, K. A., Warner, B. L. L., Proc. Natl. Acad.Sci, USA, Vol. 97, 6640-6645, (2000)] by an electroporation method to obtain a transformant (a transformant with the lacZY gene substituted with lacZY::cat-sacB) exhibiting chloramphenicol resistance and sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant without pKD46) exhibiting ampicillin sensitivity was further obtained. The transformant was named W3110S3GKΔlacZY.

Similarly, PCR was performed using, as a template, the genomic DNA of the W3110 strain and using, as a primer set, DNAs consisting of the nucleotide sequences shown in "Primer set" in Table 2 to obtain each amplified DNA fragment.

**[Table 2]**

| Primer set (SEQ ID NO:) | Amplified DNA fragment | Remarks |
|---|---|---|
| 17 and 18 | yhbJ upstream 1 | Sequences of nucleotide sequences represented by SEQ ID NOs: 9 and 17 at 5' ends are complementary |
| 19 and 20 | yhbJ downstream 1 | Sequences of nucleotide sequences represented by SEQ ID NOs: 10 and 19 at 5' ends are complementary |
| 18 and 21 | yhbJ upstream 2 | Sequences of nucleotide sequences represented by SEQ ID NOs: 21 and 22 at 5' ends are complementary |
| 20 and 22 | yhbJ downstream 2 | |

The yhbJ upstream 1 and yhbJ upstream 2 contain a region from an initiation codon of the yhbJ gene to about 1000 bp upstream of the initiation codon. The yhbJ downstream 1 and yhbJ downstream 2 contain a region from a stop codon of the yhbJ gene to about 1000 bp downstream of the stop codon.

PCR was performed using, as a template, a mixture of the yhbJ upstream 1, the yhbJ downstream 1, and the cat-sacB fragment at an equimolar ratio and using, as a primer set, DNAs consisting of the nucleotide sequences represented by SEQ ID NOs: 18 and 20 to obtain a DNA (hereinafter, referred to as yhbJ::cat-sacB) fragment consisting of a sequence with the cat-sacB fragment inserted in a region around the yhbJ gene.

PCR was performed using, as a template, a mixture of the yhbJ upstream 2 and the yhbJ downstream 2 at an equimolar ratio and using, as a primer set, DNAs consisting of the nucleotide sequences represented by SEQ ID NOs: 18 and 20 to obtain a DNA (hereinafter, referred to as ΔyhbJ) fragment consisting of a sequence with the yhbJ upstream and the yhbJ downstream directly linked to each other without yhbJ.

The yhbJ::cat-sacB fragment was introduced into the W3110S3GKΔlacZY strain constructed as described above by the electroporation method to obtain a transformant (a transformant with yhbJ substituted with yhbJ::cat-sacB) exhibiting chloramphenicol resistance and sucrose sensitivity.

The ΔyhbJ fragment was introduced into the transformant by the electroporation method to obtain a transformant (a transformant with yhbJ::cat-sacB substituted with ΔyhbJ) exhibiting chloramphenicol sensitivity and sucrose resistance. Further, a transformant (a transformant without pKD46) exhibiting ampicillin sensitivity was obtained. The transformant was named an INTC strain.

### (3) Construction of microorganism having β1,3-N-acetylglucosamine transferase activity (hereinafter referred to as LgtA activity)

Escherichia coli having a plasmid expressing a gene encoding a β1,3-N-acetylglucosamine transferase (hereinafter, referred to as NpLgtA) derived from a Neisseria polysaccharea ATCC43768 strain and consisting of the amino acid sequence represented by SEQ ID NO: 6 was constructed by the following method.

PCR was performed using, as a primer set, DNAs consisting of the nucleotide sequences shown in "Primer set" in Table 3 and using, as a template, a DNA described in "Template" in Table 3 to obtain each amplified DNA fragment.

**[Table 3]**

| Primer set (SEQ ID NO:) | Template | Amplified DNA fragment |
|---|---|---|
| 23 and 24 | DNA represented by SEQ ID NO: 5 | NplgtA |
| 25 and 26 | Genomic DNA of Escherichia coli W3110 strain | lacY |

The DNA represented by SEQ ID NO: 5 was a DNA in which a nucleotide sequence of the gene encoding a β1,3-N-acetylglucosamine transferase derived from a Neisseria polysacchareaATCC43768 strain and represented by SEQ ID NO: 6 was codon-optimized for expression in Escherichia coli and, prepared by artificial synthesis. A genomic DNA of the Escherichia coli W3110 strain was prepared by an ordinary method. The nucleotide sequences represented by SEQ ID NOs: 24 and 25 contain complementary sequences at each 5' end.

PCR was performed using, as a template, a mixture of an NplgtA fragment and a lacY fragment at an equimolar ratio and using, as a primer set, DNAs consisting of the nucleotide sequences represented by SEQ ID NOs: 23 and 26 to obtain a DNA (hereinafter, referred to as NplgtA-lacY) fragment with the two fragments linked.

PCR was performed using, as a primer set, DNAs consisting of the nucleotide sequences represented by SEQ ID NOs: 27 and 28 and using, as a template, a plasmid pUAKQE 31 (Appl. Environ. Microbiol. 2007, 73: 6378-6385) to obtain a vector fragment of about 4.7 kb.

In this case, the nucleotide sequences represented by SEQ ID NOs: 23 and 27 and SEQ ID NOs: 26 and 28 contain complementary sequences at each 5' end.

The NplgtA-lacY fragment and the vector fragment obtained above were ligated using an In-Fusion HD Cloning Kit (manufactured by Takara Bio Inc.) to obtain an NplgtA expression plasmid pLNTII.

The INTC strain constructed in Example 1 (2) was transformed using the expression plasmid pLNTII described above to construct Escherichia coli having pLNTII, which was named an INTC/pLNTII strain.

### (4) Construction of microorganism with enhanced transporter gene expression

Escherichia coli having a plasmid expressing a transporter gene derived from the W3110 strain was constructed by the following method.

PCR was performed using DNAs consisting of the nucleotide sequences shown in "Primer set" in Table 4 as a primer set and a chromosomal DNA of the W3110 strain as a template to obtain each amplified DNA fragment.

**[Table 4]**

| Primer set (SEQ ID NO:) | Amplified DNA fragment |
|---|---|
| 31 and 32 | ydeA |
| 33 and 34 | mdfA |
| 35 and 36 | setC |
| 37 and 38 | ynfM |
| 39 and 40 | mdtD |
| 41 and 42 | yfcJ |
| 43 and 44 | emrD |
| 45 and 46 | ydhC |
| 47 and 48 | ybdA |
| 49 and 50 | ydeE |
| 51 and 52 | mhpT |
| 53 and 54 | kgtP |

A chromosomal DNA of the Escherichia coli W3110 strain was prepared by an ordinary method.

PCR was performed using DNAs consisting of the nucleotide sequences represented by SEQ ID NOs: 29 and 30 as a primer set and using a plasmid pSTV29 (manufactured by Takara Bio Inc.) as a template to obtain a vector fragment of about 3 kb.

In this case, the nucleotide sequences represented by SEQ ID NOs: 31, 33, 35, 37, 39, 41, 43, 45, 47, 49, 51, 53, and 29 and SEQ ID NOs: 32, 34, 36, 38, 40, 42, 44, 46,48, 50, 52, 54, and 30 contain complementary sequences at each 5' end.

The amplified DNA fragments and the vector fragments obtained above were ligated using an In-Fusion HD Cloning Kit (manufactured by Takara Bio Inc.) to obtain 12 types of plasmids for expressing a transporter gene derived from the W3110 strain.

The INTC/pLNTII strain constructed in Example 1 (3) was transformed using the 12 types of transporter gene expression plasmids to construct 12 types of LNTII-producing strains with enhanced transporter gene expression.

### [Example 2] Production of Lacto-N-triose II (LNTII)

The INTC/pLNTII strain obtained in Example 1 and the 12 types of LNTII-producing strains with enhanced transporter gene expression were cultured on LB plates containing 100 mg/L of kanamycin at 30°C for 24 hours, inoculated into large test tubes containing 2 mL of LB culture medium containing 100 mg/L kanamycin, and cultured with shaking at 30°C for 15 hours. At this time, when culturing the LNTII strain with enhanced transporter gene expression, 25 mg/L of chloramphenicol was added to the culture medium.

Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (except for glucose, lactose monohydrate, and magnesium sulfate heptahydrate adjusted to pH 7.2 by aqueous sodium hydroxide, and then autoclaved) (aqueous solutions containing glucose, lactose monohydrate, and magnesium sulfate heptahydrate were separately prepared, autoclaved, cooled, and then mixed)] containing 100 mg/L of kanamycin, and shaking-cultured at 30°C for 29 hours. At this time, when culturing the LNTII-producing strain with enhanced transporter gene expression, 25 mg/L of chloramphenicol was added to the culture medium, and 6 hours after the start of the culture, IPTG was added to final concentration of 1 mM.

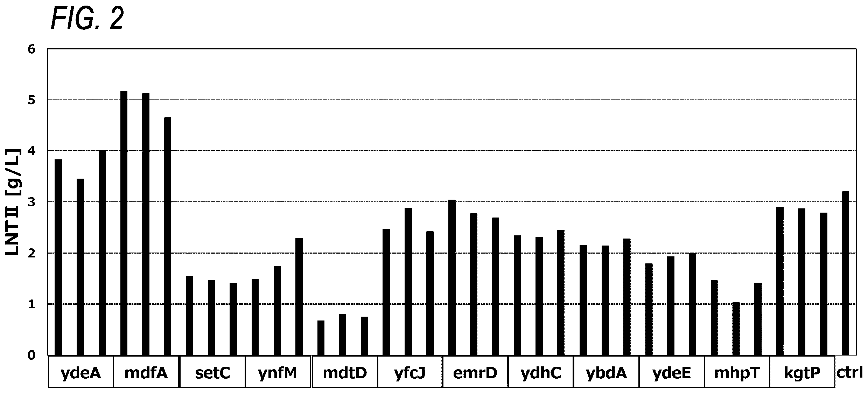

After completion of the culture, the culture solution was appropriately diluted and centrifuged, and LNTII contained in the supernatant was analyzed by a carbohydrate analyzer ICS-6000. The results are shown in FIG. 2.

As shown in FIG. 2, compared with the INTC/pLNTII strain, when expression of the ydeA gene or the mdfA gene was enhanced, significantly higher LNTII productivity was exhibited. This was suggested that the proteins encoded by the ydeA gene or the mdfA gene had an LNTII transporting activity.

Although not shown in the data, the LNTII productivity was also improved even when the expression of the setA gene and the setB gene of the W3110 strain was enhanced. This suggested that the proteins encoded by the genes also had the LNTII transporting activity.

### [Example 3] Construction of Microorganism Used in Production of Lacto-N-tetraose (LNT)

### (1) Production of Escherichia coli with deletion of ydeA Gene

PCR was performed using, as a template, a genomic DNA of the W3110 strain prepared by an ordinary method and using, as a primer set, DNAs consisting of the nucleotide sequences shown in "Primer set" in Table 5 to amplify each DNA fragment.

**[Table 5]**

| Primer set (SEQ ID NO:) | Amplified DNA fragment | Remarks |
|---|---|---|
| 55 and 56 | ydeA upstream 1 | Sequences of nucleotide sequences represented by SEQ ID NOs: 9 and 55 at 5' ends are complementary |
| 57 and 58 | ydeA downstream 1 | Sequences of nucleotide sequences represented by SEQ ID NOs: 10 and 57 at 5' ends are complementary |
| 56 and 59 | ydeA upstream 2 | Sequences of nucleotide sequences represented by SEQ ID NOs: 59 and 60 at 5' ends are complementary |
| 58 and 60 | ydeA downstream 2 | |

The ydeA upstream 1 and ydeA upstream 2 contain a region from an initiation codon of the ydeA gene to about 1000 bp upstream of the initiation codon. The ydeA downstream 1 and ydeA downstream 2 contain a region from a stop codon of the ydeA gene to about 1000 bp downstream of the stop codon.

PCR was performed using, as a template, a mixture of the ydeA upstream 1, the ydeA downstream 1, and the cat-sacB fragment obtained in Example 1 (1) at an equimolar ratio and using, as a primer set, DNAs consisting of the nucleotide sequences represented by SEQ ID NOs: 56 and 58 to obtain a DNA (hereinafter, referred to as ydeA::cat-sacB) fragment consisting of a sequence with the cat-sacB fragment inserted in a region around the ydeA gene.

PCR was performed using, as a template, a mixture of the ydeA upstream 2 and the ydeA downstream 2 at an equimolar ratio and using, as a primer set, a DNA consisting of the nucleotide sequences represented by SEQ ID NOs: 56 and 58 to obtain DNAs (hereinafter, referred to as ΔydeA) fragment consisting of a sequence with the ydeA upstream and the ydeA downstream directly linked to each other without ydeA.

Into a strain obtained by reintroducing the plasmid pKD46 into the INTC strain constructed in Example 1 (2), the ydeA::cat-sacB fragment was introduced by an electroporation method to obtain a transformant (a transformant with the ydeA gene substituted with ydeA::cat-sacB) exhibiting chloramphenicol resistance and sucrose sensitivity.

The ΔydeA fragment was introduced into the transformant by the electroporation method to obtain a transformant (a transformant with ydeA::cat-sacB substituted with ΔydeA) exhibiting chloramphenicol sensitivity and sucrose resistance. Among them, a transformant (a transformant without pKD46) exhibiting an ampicillin sensitivity was further obtained. The transformant was named an INTCΔydeA strain.

### (2) Construction of microorganism having β1,3-galactosyltransferase (GalT) activity and LgtA activity

Escherichia coli having a plasmid co-expressing a gene encoding β1,3-galactosyltransferase (hereinafter, referred to as Cvβ3GalT) derived from a Chromobacterium violaceum ATCC553 strain and consisting of the amino acid sequence represented by SEQ ID NO: 8 was constructed by the following method.

PCR was performed using DNAs consisting of the nucleotide sequences represented by SEQ ID NOs: 61 and 62 as a primer set and using the DNA represented by SEQ ID NO: 7 as a template to obtain a Cvβ3galT fragment.

The DNA represented by SEQ ID NO: 7 was prepared with reference to ACS Catal. 2019, 9 (12), 10721-10726, and was a DNA in which a nucleotide sequence of the gene encoding β1,3-galactosyltransferase derived from a Chromobacterium violaceum ATCC553 strain and represented by SEQ ID NO: 8 was codon-optimized for expression in Escherichia coli and prepared by artificial synthesis.

PCR was performed using the plasmid pLNTII obtained in Example 1 (3) as a template and using DNAs consisting of the nucleotide sequences represented by SEQ ID NOs: 27 and 63 as a primer set to obtain a vector fragment of about 7.0 kb. In this case, the nucleotide sequences represented by SEQ ID NOs: 27 and 61 and SEQ ID NOs: 62 and 63 contain complementary sequences at each 5' end.

The Cvβ3galT fragment and the vector fragment obtained above were ligated using an In-Fusion HD Cloning Kit (manufactured by Takara Bio Inc.) to obtain a Cvβ3galT and NplgtA expression plasmid pLNT.

The INTC strain constructed in Example 1 (2) and the INTCΔydeA strain constructed in Example 3 (1) were transformed using the expression plasmid pLNT described above to construct Escherichia coli having pLNT, which were named an INTC/pLNT strain and an INTCΔydeA/pLNT strain, respectively.

### [Example 4] Production of Lacto-N-tetraose (LNT)

The INTC/pLNT strain and INTCΔydeA/pLNT strain obtained in Example 3 were cultured on an LB plate containing 100 mg/L of kanamycin at 30°C for 17 hours, inoculated into a large test tube containing 2 mL of LB culture medium containing 100 mg/L kanamycin, and cultured with shaking at 30°C for 15 hours.

Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (except for glucose, lactose monohydrate, and magnesium sulfate heptahydrate adjusted to pH 7.2 by aqueous sodium hydroxide, and then autoclaved) (aqueous solutions containing glucose, lactose monohydrate, and magnesium sulfate heptahydrate were separately prepared, autoclaved, cooled, and then mixed)] containing 100 mg/L of kanamycin, and shaking-cultured at 30°C for 29 hours.

After completion of the culture, the culture solution was appropriately diluted and centrifuged, and LNTII and LNT contained in the supernatant were analyzed by a carbohydrate analyzer ICS-6000. The results are shown in Table 6.

**[Table 6]**

| Strain name | LNT (g/L) | LNTII (g/L) |
|---|---|---|
| INTC/pLNT | 2.11 | 0.51 |
| INTCΔydeA/pLNT | 2.73 | 0.63 |

As a result, the INTCΔydeA/pLNT strain exhibited LNT productivity higher compared with the INTC/pLNT strain, and it was found that the LNT productivity was improved by deleting the ydeA gene.

### [Example 5] Preparation of Aqueous Solution Containing High Purity Lacto-N-triose II (LNTII)

The pH of a culture solution obtained by culturing the Escherichia coli having an LNTII producing ability and constructed in Example 1 in a culture medium was adjusted to 3.0 using sulfuric acid, followed by heating at 70°C for 40 minutes. Subsequently, bacteria cells were removed by centrifugation (4°C, 6000 G, 7000 rpm, 10 minutes), and a supernatant was collected.

The supernatant was passed through a column filled with a cation exchange resin and a column filled with an anion exchange resin in this order to collect a fraction containing LNTII. At this time, DIAION UBK 550 (H⁺ type) (manufactured by Mitsubishi Chemical Group Corporation) as a cation exchange resin and A111S (OH⁻type) (manufactured by Prolite) as an anion exchange resin were used.

A fraction obtained using a UF membrane module (SIP-0013, manufactured by Asahi Kasei Corporation) was filtered, and the obtained filtrate was concentrated using an evaporator to obtain an LNTII-containing aqueous solution having an LNTII concentration of 800 g/L and HPLC purity of 81% (area%).

### [Example 6] Acquisition of Lacto-N-triose II (LNTII) Crystal

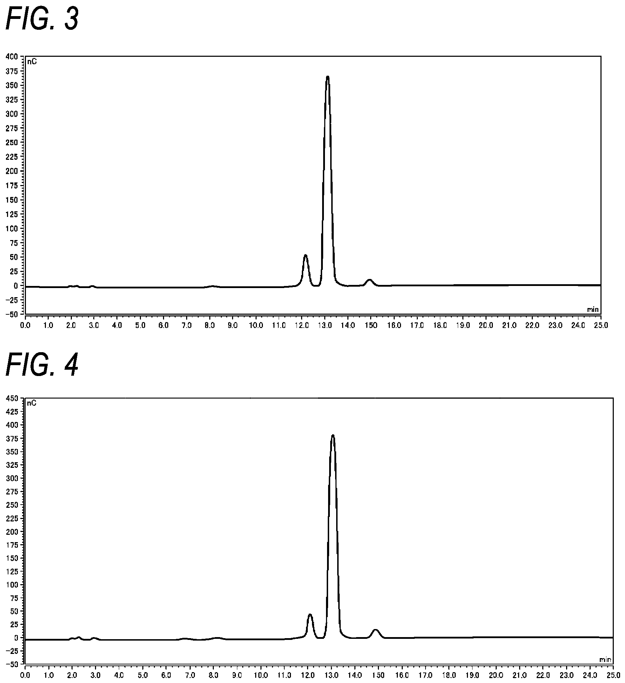

25 mL of the LNTII-containing aqueous solution obtained in Example 5 was allowed to stand at 4°C for 120 hours to precipitate a crystalline solid. The solid was collected by filtration and dried under air at room temperature for 12 hours to obtain 11.7 g of crystals. As a result of content analysis by HPLC, a content of LNTII in the obtained crystal was 80.8% (HPLC purity 84.3%). A chromatogram obtained when the crystal was analyzed by a carbohydrate analyzer ICS-6000 is shown in FIG. 3.

### [Example 7] Production of Lacto-N-triose II (LNTII) Crystal by Cooling Crystallization Method

The LNTII-containing aqueous solution obtained in Example 5 was diluted with water to adjust an LNTII concentration to 500 g/L. 36 mL of the solution was stirred while maintaining the temperature at 40°C, and the crystal obtained in Example 6 was added as a seed crystal to precipitate crystals. The crystals were aged by stirring at room temperature for 24 hours, and then further stirred at 4°C for 24 hours. Thereafter, the crystalline slurry was centrifuged using a basket type centrifuge (manufactured by KOKUSAN Co. Ltd.), and the obtained wet crystals were dried under vacuum at 30°C to obtain 2.8 g of crystals. A content of LNTII in the obtained crystal was 64.4% (HPLC purity 84.6%). A chromatogram obtained when the crystal was analyzed by a carbohydrate analyzer ICS-6000 is shown in FIG. 4.

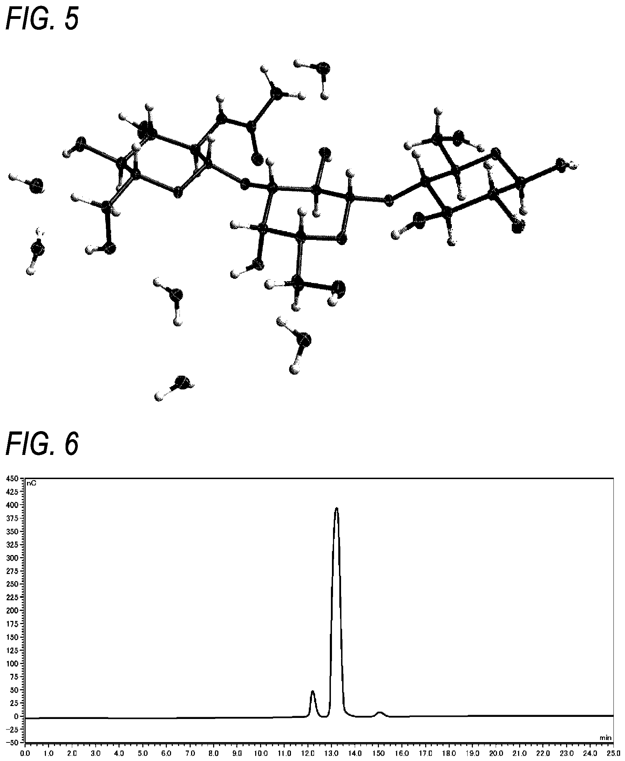

In order to determine the structure of the crystal, single crystal X-ray analysis was performed. In analysis of the obtained diffraction data, integration and absorption correction were performed using software (CrysAlis Pro, Rigaku Corporation), and then an initial structure was acquired using SHELX (Acta Crystallogr. Sect. A 2015, 71, 3-8), and the structure was refined using SHELX L (Acta Crystallogr. Sect. C 2015, 71, 3-8). The results are shown in Table 7, and the ORTEP diagram is shown in FIG. 5.

**[Table 7]**

| Crystal system | Monoclinic |
|---|---|
| Empirical formula | C₂₀H₄₇NO₂₂ |
| a (Å) | 4.64340 (10) |
| b (Å) | 27.1208 (3) |
| c (Å) | 11.77770 (10) |
| **β (°)** | 97.4430 (10) |
| V (Å³) | 1470.70 (4) |
| Measurement temperature (K) | 100.00 (10) |
| Z | 2 |
| Space group | P2₁ |
| Rint | 0.0393 |
| R₁ | 0.0330 |
| wR₂ | 0.0787 |
| GoF | 1.035 |
| Flack parameter (Parsons) | -0.036 (81) |

As a result of the analysis, it was confirmed that the crystal had a crystal structure of LNTII, and was an LNTII.6.0 hydrate having six water molecules in a unit lattice.

Subsequently, powder X-ray diffraction of the crystal was performed. Table 8 shows diffraction angles of peaks with a relative intensity ratio (I/I₀) of 10 or more based on the diffraction results. In the table, "2θ" represents a diffraction angle (2θ°), a "d value" represents a lattice spacing (dÅ), and a "relative intensity" represents a relative intensity ratio (I/I₀).

**[Table 8]**

| **2θ** | d value | Relative intensity | | **2θ** | d value | Relative intensity |
|---|---|---|---|---|---|---|
| 8.2 | 10.826 | 66 | | 23.1 | 3.854 | 44 |
| 9.9 | 8.927 | 29 | | 23.6 | 3.773 | 41 |
| 15.0 | 5.917 | 17 | | 25.0 | 3.565 | 26 |
| 16.4 | 5.407 | 11 | | 26.1 | 3.417 | 27 |
| 17.9 | 4.957 | 21 | | 27.4 | 3.255 | 22 |
| 19.3 | 4.600 | 100 | | 30.2 | 2.959 | 25 |
| 19.6 | 4.535 | 88 | | 32.7 | 2.736 | 22 |
| 19.9 | 4.467 | 51 | | 38.3 | 2.346 | 20 |
| 21.4 | 4.156 | 37 | | 38.5 | 2.338 | 20 |
| 22.2 | 3.994 | 26 | | 39.5 | 2.282 | 16 |

### [Example 8] Production of Lacto-N-triose II (LNTII) Crystal by Poor Solvent Addition Method

The LNTII-containing aqueous solution obtained in Example 5 was used to prepare a solution with an LNTII concentration of 400 g/L. While stirring 40 mL of the solution at 40°C, 60 mL of ethanol was added dropwise as a poor solvent. The crystal obtained in Example 6 was added as a seed crystal to precipitate crystals. The crystals were aged by stirring at room temperature for 72 hours, and then further stirred at 4°C for 24 hours. Thereafter, the crystalline slurry was centrifuged by a basket type centrifuge to obtain 16.1 g of wet crystals. The obtained wet crystals were dried under vacuum at 30°C to obtain 7.2 g of crystals. A content of LNTII in the crystal was 72.9% (HPLC purity 89.7%). A chromatogram obtained when the crystal was analyzed by a carbohydrate analyzer ICS-6000 is shown in FIG. 6.

Table 9 shows diffraction angles of peaks with a relative intensity ratio (I/I₀) of 10 or more based on the results of powder X-ray diffraction of the obtained crystals. In the table, "2θ" represents a diffraction angle (2θ°), a "d value" represents a lattice spacing (dÅ), and a "relative intensity" represents a relative intensity ratio (I/I₀).

**[Table 9]**

| **2θ** | d value | Relative intensity | | **2θ** | d value | Relative intensity |
|---|---|---|---|---|---|---|
| 4.7 | 18.627 | 13 | | 25.0 | 3.553 | 24 |
| 8.3 | 10.695 | 42 | | 25.5 | 3.488 | 22 |
| 9.5 | 9.263 | 92 | | 26.9 | 3.314 | 62 |
| 12.7 | 6.986 | 44 | | 28.6 | 3.119 | 23 |
| 14.4 | 6.163 | 11 | | 29.4 | 3.034 | 41 |
| 16.6 | 5.336 | 31 | | 30.2 | 2.955 | 14 |
| 17.3 | 5.122 | 19 | | 31.8 | 2.813 | 27 |
| 18.6 | 4.772 | 100 | | 34.0 | 2.635 | 30 |
| 19.2 | 4.624 | 28 | | 35.0 | 2.559 | 11 |
| 19.8 | 4.484 | 94 | | 36.8 | 2.442 | 20 |
| 20.9 | 4.239 | 65 | | 37.4 | 2.401 | 17 |
| 21.5 | 4.134 | 64 | | 38.1 | 2.361 | 18 |
| 22.0 | 4.030 | 47 | | 40.0 | 2.252 | 16 |
| 23.1 | 3.854 | 25 | | 40.6 | 2.219 | 18 |
| 23.6 | 3.770 | 33 | | 47.0 | 1.933 | 18 |
| 24.1 | 3.696 | 30 | | | | |

From the above results, it was confirmed that the LNTII crystal obtained by the poor solvent addition method described in Example 8 was the same as the crystal described in Patent Literature 4.

Although the present invention has been described in detail with reference to specific embodiments, it will be apparent to those skilled in the art that various changes and modifications can be made without departing from the spirit and scope of the present invention. The present application is based on a Japanese patent application filed on December 21, 2021 (patent application No. 2021-207590), the entire contents of which are incorporated herein by reference.

### Sequence Listing Free Text

SEQ ID NO: 1: nucleotide sequence of ydeA derived from E. coli W3110
SEQ ID NO: 2: amino acid sequence of ydeA derived from E. coli W3110
SEQ ID NO: 3: nucleotide sequence of mdfA derived from E. coli W3110
SEQ ID NO: 4: amino acid sequence of mdfA derived from E. coli W3110
SEQ ID NO: 5: nucleotide sequence of codon optimized NplgtA
SEQ ID NO: 6: amino acid sequence of NpLgtA
SEQ ID NO: 7: nucleotide sequence of codon optimized Cvβ3galT
SEQ ID NO: 8: amino acid sequence of Cvβ3GalT
SEQ ID NOs: 9 and 10: nucleotide sequence of catsacB fragment amplification primer
SEQ ID NOs: 11 and 12: nucleotide sequence of lacZ upstream 1 amplification primer
SEQ ID NOs: 13 and 14: nucleotide sequence of lacY downstream 1 amplification primer
SEQ ID NO: 15: nucleotide sequence of lacZ upstream 2 amplification primer
SEQ ID NO: 16: nucleotide sequence of lacY downstream 2 amplification primer
SEQ ID NOs: 17 and 18: nucleotide sequence of yhbJ upstream 1 amplification primer
SEQ ID NOs: 19 and 20: nucleotide sequence of yhbJ downstream 1 amplification primer
SEQ ID NO: 21: nucleotide sequence of yhbJ upstream 2 amplification primer
SEQ ID NO: 22: nucleotide sequence of yhbJ downstream 2 amplification primer
SEQ ID NOs: 23 and 24: nucleotide sequence of NplgtA fragment amplification primer
SEQ ID NOs: 25 and 26: nucleotide sequence of lacY fragment amplification primer
SEQ ID NOs: 27 and 28: nucleotide sequence of pUAKQE31 amplification primer
SEQ ID NOs: 29 and 30: nucleotide sequence of pSTV29 amplification primer
SEQ ID NOs: 31 and 32: nucleotide sequence of ydeA fragment amplification primer
SEQ ID NOs: 33 and 34: nucleotide sequence of mdfA fragment amplification primer
SEQ ID NOs: 35 and 36: nucleotide sequence of setC fragment amplification primer
SEQ ID NOs: 37 and 38: nucleotide sequence of ynfM fragment amplification primer
SEQ ID NOs: 39 and 40: nucleotide sequence of mdtD fragment amplification primer
SEQ ID NOs: 41 and 42: nucleotide sequence of yfcJ fragment amplification primer
SEQ ID NOs: 43 and 44: nucleotide sequence of emrD fragment amplification primer
SEQ ID NOs: 45 and 46: nucleotide sequence of ydhC fragment amplification primer
SEQ ID NOs: 47 and 48: nucleotide sequence of ybdA fragment amplification primer
SEQ ID NOs: 49 and 50: nucleotide sequence of ydeE fragment amplification primer
SEQ ID NOs: 51 and 52: nucleotide sequence of mhpT fragment amplification primer
SEQ ID NOs: 53 and 54: nucleotide sequence of kgtP fragment amplification primer
SEQ ID NOs: 55 and 56: nucleotide sequence of ydeA upstream 1 amplification primer
SEQ ID NOs: 57 and 58: nucleotide sequence of ydeA downstream 1 amplification primer
SEQ ID NO: 59: nucleotide sequence of ydeA upstream 2 amplification primer
SEQ ID NO: 60: nucleotide sequence of ydeA downstream 2 amplification primer
SEQ ID NOs: 61 and 62: nucleotide sequence of Cvβ3galT fragment amplification primer
SEQ ID NO: 63: pLNTII fragment amplification primer
SEQ ID NO: 64: nucleotide sequence of setA derived from E. coli W3110
SEQ ID NO: 65: amino acid sequence of setA derived from E. coli W3110
SEQ ID NO: 66: nucleotide sequence of setB derived from E. coli W3110
SEQ ID NO: 67: amino acid sequence of setB derived from E. coli W3110

## Claims

1. A microorganism having an enhanced activity of a protein according to any one of the following [1] to [3] and having improved productivity of lacto-N-triose II (LNTII) as compared with a parent strain:
[1] a protein consisting of the amino acid sequence represented by SEQ ID NO: 2 or 4,
[2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
[3] a homologous protein having an oligosaccharide transporting activity and consisting of an amino acid sequence having an identity of 90% or more with the amino acid sequence represented by SEQ ID NO: 2 or 4.

2. A microorganism having a reduced or inactivated activity of a protein according to any one of the following [1] to [3] and improved productivity of an oligosaccharide having a lacto-N-triose II (LNTII) skeleton as compared with a parent strain:
[1] a protein consisting of the amino acid sequence represented by SEQ ID NO: 2 or 4,
[2] a mutant protein having an oligosaccharide transporting activity and consisting of an amino acid sequence in which 1 to 20 amino acids are deleted, substituted, inserted, or added in the amino acid sequence represented by SEQ ID NO: 2 or 4, and
[3] a homologous protein having an oligosaccharide transporting activity and consisting of an amino acid sequence having an identity of 90% or more with the amino acid sequence represented by SEQ ID NO: 2 or 4.

3. The microorganism according to claim 2, wherein
the oligosaccharide having the LNTII skeleton is lacto-N-tetraose (LNT) or lacto-N-neotetraose (LNnT).

4. A method for producing an oligosaccharide, the method comprising:
preparing the microorganism according to any one of claims 1 to 3; and
producing an oligosaccharide in a culture using the microorganism.

5. A method for producing an LNTII crystal from a culture obtained by culturing the microorganism according to claim 1, the method comprising the following steps (i) to (iv):
(i) a step of centrifuging the culture to obtain a supernatant from which bacteria cells are removed;
(ii) a step of subjecting the supernatant obtained in the step (i) to cation exchange and anion exchange to obtain a solution from which ions are removed;
(iii) a step of concentrating the solution obtained in the step (ii) with an evaporator; and
(iv) a step of cooling the solution obtained in the step (iii) or adding dropwise a poor solvent to the solution obtained in the step (iii) to obtain an LNTII crystal.
